(19) Europäisches Patentamt
European Patent Office
Office européen des brevets

(11) Publication number: **0 091 740**
**A2**

(12) # EUROPEAN PATENT APPLICATION

(21) Application number: 83301515.9

(22) Date of filing: 18.03.83

(51) Int. Cl.$^3$: **A 61 K 7/09**

(30) Priority: 19.03.82 US 359645

(43) Date of publication of application: 19.10.83 Bulletin 83/42

(84) Designated Contracting States: AT BE CH DE FR GB IT LI LU NL SE

(71) Applicant: SYNTEX (U.S.A.) INC.
3401 Hillview Avenue
Palo Alto, California 94304(US)

(72) Inventor: Lindentahl, Margaret G.
1417 Village Court
Mt. View California 94040(US)

(72) Inventor: Edelstein, Herbert
1178 Hyde Avenue
San Jose California 95129(US)

(74) Representative: Armitage, Ian Michael et al,
MEWBURN ELLIS & CO. 2/3 Cursitor Street
London EC4A 1BQ(GB)

(54) **A composition and process for treating hair.**

(57) The composition comprises a water soluble bisulfite salt, a cationic surfactant penetrating agent, unsubstituted or substituted imidazolidinedione, and a water soluble organic acid salt buffered to a pH between 5.0-6.5. The process for treating hair comprises applying said composition to the hair, shaping the hair and, after an appropriate time, rinsing the hair with water.

EP 0 091 740 A2

Croydon Printing Company Ltd.

# A COMPOSITION AND PROCESS FOR TREATING HAIR

## BACKGROUND OF THE INVENTION

### Field of the Invention

This invention relates to a composition and process for imparting a permanent set to hair. More particularly it relates to a buffered bisulfite, cationic surfactant, unsubstituted or substituted imidazolidinedione and water soluble organic acid salt containing composition for waving or straightening hair requiring only water rinsing to complete the hair treating process.

### Related Art

Hair waving requires the breaking, rearranging and reforming of keratin protein bonds which are responsible for hair configuration such as covalent disulfide bonds, ionic bonds between adjacent ionized amino acids of different polarity, and short range polar bonds, e.g. hydrogen bonds. Rearranging ionic and hydrogen bonds can be quickly done with water, wet heat or both in conjunction with mechanical shaping devices. Unfortunately these methods produce only short term results. Permanent hair shaping can best be achieved by breaking, physically rearranging and reforming the cystine disulfide bond (-S-S-) linkages prevalent in keratin protein.

Affecting natural hair shape by lysing the cystine

disulfide linkage is referred to as softening or relaxing the hair. Softening can be done by chemical reduction or with steam. Chemical reduction is usually preferred. Once the hair has been softened, or during the softening step, the hair can be artificially shaped to a desired configuration. Reducing chemicals are then neutralized or washed from the hair, followed generally by an oxidation step to regenerate disulfide linkages between the same or previously unconnected cysteine moieties. Reformation of cystine disulfide bonds or other sulfur-based intrachain protein linkages is sometimes referred to as "hardening." These new cystine disulfide bonds permanently maintain the hair in the shape it was given during the softening and hardening steps.

Hair softening chemicals are generally called hair waving agents, though the same agents may be used to straighten hair as well. At present, one of the most widely used types of hair waving and straightening compositions are the sulfur-based cold wave solutions. Their name is derived from the fact they can provide good wave or straightening action at low (room) or moderate temperatures rather than requiring the use of steam or high temperatures during the softening process.

Almost all cold wave solutions contain a reducing agent comprising a sulfur-containing chemical, most commonly thiol substituted acids, aldehydes and alcohols; or bisulfite salts and sulfites. A conventional method for utilizing such solutions is to treat the hair with a solution containing one of these reductive ingredients at alkaline pH, forming the hair into the desired configuration and then treating it with a so-called neutralizing solution or an oxidizing reagent such as a bromate, perborate, or hydrogen peroxide to effect reformation of sulfide bonds. This method enables

satisfactory permanent hair shapes to be formed at room temperature.

Among the thiol substituted compounds, thioglycolic acid salts are the most frequently used cold waving agents. However, these particular acid salts or other thiol acid salts, even though they can be used at low temperature, exhibit several problems from the standpoint of hygiene and safety even when used under strictly controlled conditions and in optimized amounts. Waving solutions based on thioglycolic acid frequently emit a strong unpleasant sulfur-based odor due to mercaptan compounds. This odor is a nuisance to cosmetologists and users. Also, thioglycolic acid salt-based waving solutions only provide good cold wave permanents at a pH of 8.0 or above, which requires the use of an alkaline substance to adjust pH. However, the adjustment of pH, usually done with ammonia, increases the unpleasantness of the sulfur-based odor and the ammonia, or any other base, may cause irritation and sensitization upon contact with the skin.

Alternate room temperature-active hair softening compositions based on the sulfite or bisulfite moiety are known. Sulfite and bisulfite-based solutions exhibit lower acute toxicity and lessened reaction with human skin in relation to thioglycolic acid-based compositions.

An early U.S. patent to J. Speakman, number 2,201,929, discloses the use of sulfites as softening agents in solutions at pHs ranging from 4 through 11, preferably at pH 11. Variations of Speakman's original sulfite and bisulfite cold wave solutions have been proposed. For example, there is a British Patent No. 591,936 to J. Speakman disclosing the addition of 15% to 45% alcohol to such sulfite and bisulfite cold wave lotions. The replacement of alkali sulfites and bisulfites with the sulfites of organic bases, such as

monoethanolamine, ethylenediamine, and morpholine are disclosed in U.S. Patent No. 2,437,965 to E. Michaels. In addition, sulfite-based cold wave lotions containing sulfocyanate and diethylene glycol ethers are disclosed in French Patent No. 1,076,766 to J. Scandel; bisulfites and urea and urea derivatives, for example, thiourea, formamide, acetamide, di and triacetamine are disclosed in French Patent No. 1,039,222; and different glycol ethers such as Cellosolve, Carbitol, dioxane, and trioxane in combination with sulfite and bisulfite are disclosed in French Patent No. 1,073,465. These various additives are all acclaimed to intensify the hair setting action of the sulfite/bisulfite combination. Each is most effective at a pH above 6; and requires the use of some user-applied neutralizing or oxidizing chemical other than water as an after-treatment.

Other bisulfite-based hair setting compositions have been proposed, some requiring no special after-treatment other than a water rinse. For example U.S. Patent No. 3,966,903 discloses a hair waving composition which comprises a sulfite and bisulfite waving agent and an alkylene carbonate or alpha or delta lactone accelerating agent requiring only water rinsing to effect the hair set. These solutions are effective in a pH range from 6 to 9. U.S. Patent 4,038,959 discloses a hair treating composition comprising a bisulfite salt and a mink oil based quaternary ammonium salt conditioning agent. This composition is effective below pH 7, preferably 6.5-6.9. A neutralization step is optional. In another instance, a hair shampoo for increasing hair body is disclosed in U.S. Patent No. 4,243,659. This composition comprises a bisulfite salt, a urea-derived swelling agent, an auxiliary swelling agent which is a polyhydroxy alkyl solvent and a cationic hair conditioner adjusted to a pH between 4.0 to 6.9, preferably 6.0 to 6.6. See also U.S.

Patent 3,912,808 and U.S. Patent 2,836,185 which disclose bisulfite reducing agents for treating hair in combination with amine polymers and swelling agents such as water-soluble nitrogen containing compounds of the amide class. U.S. Patent 3,227,615 discloses the use of water-soluble bisulfite and a water-soluble cationic polyamide-epichlorohydrin resin for the permanent waving of hair.

A monograph on sulfite cold waving and hair straightening, authored by W. R. Markland, can be found in Norda Briefs, No. 493, July/August 1979.

All these references disclose systems which include a second reducing chemical and/or recommend, with the exception of U.S. Patent 4,038,995, the use of a neutralizing or oxidizing agent as an after-treatment in order to achieve an acceptable permanent wave. While these systems work well, the ideal and most convenient hair waving system would be one which requires only the application of a reducing composition buffered to the pH of hair, shaping of the hair, application of low to moderate heat, and water rinsing to effectuate the set. It is desirable therefore to provide a hair treating composition which is optimally active at the normal pH of hair, is effective at low to moderate temperatures and does not require the subsequent application of a neutralizing or oxidizing solution.

SUMMARY OF THE INVENTION

One aspect of this invention is an aqueous hair treating composition which comprises a water-soluble bisulfite salt, a water-soluble alkyl substituted betaine or quaternized amido amine derived cationic penetrating agent, a water-soluble organic acid salt and an imidazolidinedione having the formula:

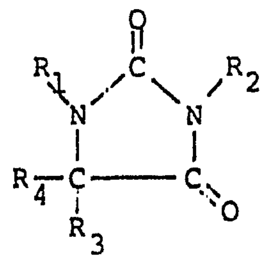

wherein $R_1$ and $R_2$ are the same or different and are hydrogen or hydroxyalkyl of 1 to 4 carbon atoms; $R_3$ and $R_4$ are the same or different and are hydrogen, alkyl of 1 to 4 carbon atoms or phenyl; and the cosmetologically acceptable acid addition salts thereof. The composition is maintained at a pH between 5 and 6.5. Optionally, buffering agents or antioxidents may be included.

Another aspect of this invention is a process for treating hair which process comprises applying the above described composition to hair for a time sufficient to effect hair treatment with low to moderate heat and then rinsing the hair with water.

There is disclosed herein a hair treating composition which gives unique and surprising hair treating results. The optimum activity of these compositions occurs at the normal pH of hair. Low to moderate temperatures will readily effect the reduction process. No after-treatment other than a water rinse is required to effect permanent hair treatment. Hair may be waved or straightened by applying this composition to hair, shaping the hair either by curling or straightening while applying low to moderate heat, and after an appropriate period of time, rinsing the hair with water. No further treatment is needed or required to impart a suitably long-lasting and manageable permanent shape to the hair.

For the purposes of this invention the phrase "hair treating" should be understood to include curling or waving, straightening, training and any other treatment which gives the hair desired configuration by means of treatment with the subject compositions in accordance with the process discussed below. "Permanent" refers here to the situation where the hair retains its post-treatment configuration even after a large number of washings or wettings with non-reductive materials.

Water-soluble bisulfite salts are the reducing agents in these unique compositions. They are convenient and safe chemical agents for reducing cystine disulfide linkages and are active at low to moderate temperatures, which in this instance refers to temperatures which vary between about 25° to 50°C. They are essentially odorless and can be formulated into stable systems at an acid pH. In proper concentrations, the subject bisulfite salts do not exhibit dermatological toxicity or sensitization.

The full range of water-soluble bisulfite salts may be used in the practice of this invention. For the purposes of this invention it is most convenient, and therefore preferred, to use a sodium, potassium, ammonium or alkanolamine salts, for example, mono, di or triethanolamine bisulfites. Sodium bisulfite is the most preferred reducing agent herein. Alkali metal metabisulfites, which hydrolyze to bisulfites in water, may also be used though they are not preferred over sodium bisulfite. The anhydrous form of sodium bisulfite usually is supplied as sodium metabisulfite. The instant compositions will contain between 5.0 and 15.0 weight/weight percent of bisulfite salt, preferably in an amount of 10%. These figures apply to all water-soluble bisulfite salts having utility herein regardless of the cation.

A second component of this composition is a

water-soluble organic acid salt. A "water-soluble organic acid salt" means the salt of a carboxylic acid function-containing hydrocarbon derived compound which is sufficiently water-soluble to give a functional concentration and which, at the concentration employed, exhibits no untoward or deleterious dermatological effects.

The presence of such salt surprisingly has been found to materially and substantially enchance the hair treating activity of the composition. By adding an appropriate amount of such salt, the bisulfite salt-based composition is rendered effective in a pH range lower than previously practiced with bisulfite salt-based hair treating compositions. Further, this salt's presence reduces treatment time and temperature. Hair texture, feel and strength is also enchanced by addition of this salt. Without this salt, a bisulfite salt or bisulfite salt/cationic penetrating agent hair treating composition does not provide the long lasting permanent or acceptable hair qualities seen when the salt is present.

Water soluble organic acid salts in general will function in this invention so long as they have the requisite water solubility and are non-toxic. Water solubility will vary depending on the organic acid selected and the counter ion. While there may be instances where one salt form will not demonstrate the requisite solubility, such will not foreclose the use of other salts of that acid. Additionally, while preferred and most preferred concentration ranges are set out infra, a salt need not be water-soluble over the full concentration range in order to come within the scope of this invention. It will be sufficient for this invention if a particular salt is water-soluble at the lower limit of the preferred concentration range.

For the purposes of this invention, water-soluble organic acid salts include the salts of simple organic acics such as the formates, acetates, propionates and the like; di or triacid salts, such as oxalates, malonates, succinates and citrates; mono or polyhydroxy substituted organic acid salts, for example glycolates, lactates, citrates, glucoronates, ascorbates and the like; and aryl acid salts such as benzoates. This is an exemplary list of organic acid salts and it should not be taken as limiting of the organic acid salts which can be used in the practice of this invention. Only water solublity and cosmetological toxicity are limiting herein.

The cation may be a metal, an amine or an alkyl or alkanolamine or the like, so long as the salt has the requisite water solubility. Acid salts will preferably be the sodium, potassium or ammonium salts. For the practice of this invention it is preferred to use an acetate salt, most preferably sodium acetate. Organic acid salt concentrations may vary between 0.5 and 20.0 weight/weight percent (w/w), most preferably in an amount between 1.0% and 10%.

The composition of this invention also contains a water-soluble alkyl substituted betaine or quaternized amido amine based surfactant. While numerous cationic surfactants are known to act as penetrating agents, it has been found that only the described compounds are efficacious in combination with the other two components. Anionic, nonionic, amphoteric and the like were found to have little or no effect on the hair treating activity of the water-soluble bisulfite/organic acid salt composition.

The general formula for the alkyl substituted betaine surfactant is

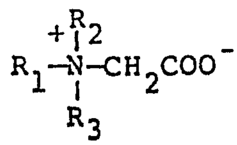 (A)

wherein $R_1$ is a saturated or unsaturated long chain fatty acid radical of 12 to 20 carbon atoms and $R_2$ and $R_3$ represent a short straight or branched chain alkyl or hydroxyalkyl radicals of 1 to 4 carbon atoms. The most preferred surfactant of this type is the one according to Formula A wherein $R_2$ and $R_3$ are both hydroxyethyl and $R_1$ is derived from the fatty acid complex called tallow.

Tallow, a U.S. designation, is the fat from fatty tissue of cattle or sheep. The highest grade of beef tallow is called oleo tallow. It contains (as glycerides): oleic acid (37-43%), palmitic acid (24-32%), stearic acid (20-25%), myristic acid (3-6%), linoleic acid (2-3%) with additional minor constituents such as cholesterol, arachidonic, elaidic, and vaccenic acids.

This preferred surfactant is designated bis-(2-hydroxyethyl) tallow ammonium ethanoate or, by the CTFA dictionary, 2nd Ed. (1978), dihydroxyethyl tallow glycinate. It is commercially available from Miranol Chemical Co., Inc., South Brunswick, New Jersey, 08810.

Alternatively, there may be used a water-soluble quaternary amido amine of Formula B

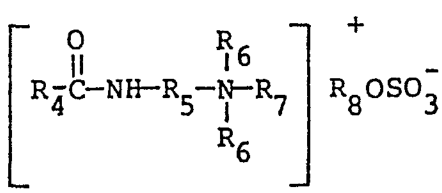 (B)

wherein $R_4$ is a saturated or unsaturated fatty acid hydrocarbon radical of 12 to 20 carbons and $R_5$ through $R_8$ are alkyl radicals of 1 to 4 carbon atoms or hydrogen.

Most preferred is the compound according to Formula B

wherein $R_4$ is the isostearyl fatty acid radical, $R_5$ is propyl, $R_6$ is methyl and $R_7$ and $R_8$ are ethyl, being the compound N,N-dimethyl-N-ethyl-N-(3-isostearyl) amidopropyl ammonium ethyl sulfate. Its CTFA dictionary, 2nd Ed. (1978), designation is isostearamidopropyl ethyl dimonium ethosulfate. Such material is available from the Scher Chemical Co., Inc., Clifton, New Jersey, 07012.

These penetrating agents may be used alone or in combination. The total amount present in these compositions will range between 0.1 and 3.0 weight/weight percent (w/w). Preferably the total amount of penetrating agent will be 0.25 to 1.5% (w/w).

A fourth ingredient herein are unsubstituted or substituted imidazolidinediones of the general formula:

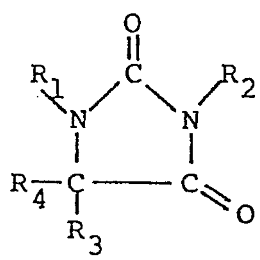

wherein $R_1$ and $R_2$ are the same or different and are hydrogen or hydroxyalkyl of 1 to 4 carbon atoms; $R_3$ and $R_4$ are the same or different and are hydrogen, alkyl of 1 to 4 carbon atoms or phenyl; and the cosmetologically acceptable acid addition salts thereof. It has been found, surprisingly, that these imidazolidinediones enhance the speed of the hair treating action, and decrease the temperature at which the hair treating process needs to be carried out. In addition, inclusion of these imidazolidinediones improves the permanence of the hair set.

For the purposes of this invention an alkyl chain of 1 to 4 carbon atoms denotes a straight chain, saturated

alkyl radical exemplified by the group methyl, ethyl, n-propyl, iso-propyl, n-butyl or t-butyl.

"Cosmetologically acceptable acid addition salts" are those salts which may be prepared by treating the subject imidazolidinediones with an acid to form a salt which retains the activity of the parent compound and exhibits no untoward or deleterious effects on the hair or skin. Such salts are those formed with, for example, inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, salicylic acid and the like.

Some of these imidazolidinediones are also available as polymeric gels, having been polymerized with formaldehyde. When mixed with water, these polymers depolymerize to yield formaldehyde and the imidazolidinedione. Such materials are within the scope of this invention.

The instant compounds are readily available from commercial sources such as Glyco Chemicals, Inc., Box 700, 51 Weaver Street, Greenwich, CT, 06830 or may be prepared by methods well known in the art. For example, the unsubstituted diones may be prepared by methods set out in U.S. Pat. No. 2,663,713. Those compounds where $R_1$ and $R_2$ are hydrogen and $R_3$ and $R_4$ are alkyl of 1 to 4 carbon atoms or phenyl may be prepared by several methods as exemplified in U.S. Pat. No.s 2,409,754; 2,402,754; 2,663,713 or from such literature references as P. Karrer, Organic Chemistry, Elsevier Pub. Co., 4th Ed., pp 276 and 296 (1950) and the American Chemical Society

Monograph No. 150, Industrial Organic Nitrogen Compounds, M. J. Astle, Reinhold Pub. Co., p 271, (1961). Hydroxyalkyl substituted diones can be made by methods such as those set out in German Patent Applications (OLS) DE 1912026, DE 2456078 and DE 2357936; U.S. Pat. 3,987,184 and Swiss Pat. 521970, to name a few.

Preferred imidazolidinediones are those wherein $R_3$ and $R_4$ are hydrogen or methyl and $R_1$ and $R_2$ are unsubstituted or substituted with hydroxyalkyl of 1 to 3 carbon atoms.

The most preferred imidazolidinediones herein are 1,3-dihydroxymethyl-5,5-dimethyl-2,4-imidazolidinedione; 1,3-dihydroxyethyl-5,5-dimethyl-2,4-imidazolidinedione; 5,5-dimethyl-2,4-imidazolidinedione; and 1-hydroxymethyl-5,5-dimethyl-2,4-imidazolidinedione.

These imidazolidinediones may be used alone or as a mixture but it is preferred to use only one dione per composition formulation. One or more compounds may be present in a cumulative amount of between 0.01% and 5% by weight/weight of the solution. Preferably the dione will present in a total amount of between 0.1% and 3% (w/w).

These compositions are adjusted to a pH of between 5 and 6.5, preferably between 5.3 - 5.5. It is important to maintain the pH of these compositions within a range of 5 to 6.5 for several reasons. First this range maximizes the reducing capacity of the composition. Secondly this range stabilizes the composition; and thirdly the normal pH of hair falls in this range.

Bisulfite ion is the most effective cystine disulfide bond reducing agent vis-a-vis the sulfite ion. Below pH 6 bisulfite ion is the only species present while above that pH the solution represents an equilibrium between bisulfite and sulfite ion.

In another instance, stability considerations indicate it is desirable to maintain this pH range

because below pH 5 the compositions, after several days at room temperature, gives off noxious fumes and shows a concurrent decrease in activity. But above 5 the compositions remain stable and do not loose their potency.

Since the normal pH range of hair is 5.3 - 5.5, it is most desirable to treat hair at this pH in order to minimize adverse affects to the hair. This pH range also is less likely to sensitize or irritate the scalp or other skin the composition may contact.

The instant aqueous solutions as formulated will generally have a neutral to acid pH, being usually within or very close to the preferred range. However, it is best to monitor the pH during manufacturing and make an appropriate adjustment with acid or base as the last formulation step.

If a pH adjustment is needed, a dilute solution of acid or base is best used. Any composition compatible acid or base may be used for this purpose. If the pH must be lowered it is preferred to use the acid form of the organic acid salt for this purpose. Where the pH must be adjusted upward, the most frequent case, ammonium hydroxide or an alkanolamine are the preferred alkali materials. Other nitrogen derived bases, alkaline earth metal hydroxides and the like will serve this purpose equally well. It is the adjustment of pH which is of significance here, not the acid or alkali used.

Although not required, it is suggested to include a buffering agent in these compositions to stabilize the pH within the preferred range. Such materials, preferably weak acids and their salts, must have some buffering capability in the preferred pH range and be compatible with other composition materials. Depending on the chosen water-soluble organic acid salt, that salt can be incorporated into the composition as one of the buffering agent components. An acetate salt/acetic acid buffer

system is exemplified hereinafter and is the preferred buffering agent for this invention where such is employed, but other weak acid based systems with buffering capability in the preferred pH range will have equal utility.

The makeup of added buffers will depend on the particular conjugate acid-base system chosen and on the buffering capacity thought to be necessary. Determination of the ratio of molar concentrations required to maintain the pH range set out herein can be conveniently calculated by known mathematical equations, the Henderson-Hasselbach equation for example. Because the instant compositions are composed of salts or weakly acidic components, buffering capacity need not be great. If capacity is of concern it can be readily calculated by means of the Van Slyke equation for determining buffer capacity.

A preferred buffer system herein is an acetic acid/sodium acetate system prepared with a ratio of molar concentrations such that the buffer pH before addition to the formulation falls around pH 5.5 or there abouts. That ratio is about 1 part acetic acid for every 7.7 parts sodium acetate. An exemplary buffer would be a 0.35 M acetic acid and 2.68 M sodium acetate solution. A hair treating composition according to the teachings of this invention and containing this buffer in an amount of about 10% is adequately buffered. This buffer and the specific figures given here are representative of the buffer systems which can be used in the practice of this invention and are not limitative in any manner thereof.

Antioxidants may be added to these compositions if desired. They will serve to improve the composition's stability and can indirectly assist in maintaining pH. Any suitable compatible antioxidant useful in pharmaceutical or cosmetological compositions can be

used. Erythorbic acid and ascorbic acid for example, can be used for this purpose. An antioxidant will be present in an amount of about 0.1 to 0.5 weight/weight percent.

Further, if it is desired, perfumes and the like may be added to these compositions so long as they do not adversely affect the hair treating activity of the composition.

The process for treating hair according to this invention comprises applying the subject compositions to hair by some convenient means, shaping the hair and resaturating it with solution if desired, followed by the application of low to moderate heat for some time sufficient to effect hair treatment. Following these steps, a water rinse will complete the setting process.

The amounts of solution required to effect hair treatment is not exact. However the hair should be well wetted, if not saturated, in order to realize to the fullest extent the hair treating activity of these compositions. If the hair is not fully wetted disulfide bond reduction will not be maximized, which can result in less stable permanents.

These compositions can be applied to dry or wet hair but it is preferred to first wash the hair and then apply the instant compositions while the hair is still wet. The compositions can be applied as a spray, as droplets or placed on some mechanical device such as a comb and applied to the hair thereby. Generally a more even distribution of the composition can be achieved by applying the composition to a comb and combing it into the hair; or combing the hair after the composition has been applied as a spray or droplets.

While it is usual to apply such solutions before shaping the hair, to insure complete and adequate wetting, solution may be reapplied once the hair is on mandrels. After the hair has been wetted and shaped a

plastic bag or some other non-porous material may be placed over the hair to keep it moist during the treatment period at the option of the practitioner.

Heat is not required to effect a good permanent wave with these compositions but the use of moderate temperatures will expedite the process. A preferred practice of this invention will include the application of some heat to the hair after it has been wetted. Temperatures should not exceed 50°C. A temperature less than this is more than adequate to insure proper and complete hair treatment. Preferably a temperature of 40° to 45°C will be used.

The amount of time for effecting hair treatment is that time which is sufficient to effect a long lasting permanent hair set. Because the proper amount of time will depend on the hair type, condition of the hair, the degree of hair wetting with the subject compositions, and the processing temperature, only a general time range can be specified. The exact time will be determined by the user based on his or her skill and knowledge of the several noted factors.

It has been found that, generally, the hair treating process will not require more than 60 minutes and usually is completed in 15 to 30 minutes, especially when moderate heat is applied. However it should be understood that because hair treatment is dependent on a variety of factors, for a given head of hair, a good permanent may be realized in less than 15 minutes.

Following the reduction step, hair need only be rinsed with running water and dried to effect a set to the hair. Usually the hair will be rinsed while still on mandrels or other shaping device. Several minutes of thorough water rinsing, about 5 minutes or so, generally removes all residual bisulfite/organic acid/surfactant material. The hair can then be removed from the shaping

device and dried or, for a more curly hair shape, dried on the shaping devices, at the choice of the user.

The following examples further illustrate the present invention.

EXAMPLE 1

A hair treating composition was prepared by conventional procedures, comprising:

TABLE I

| Ingredient | Amounts |
|---|---|
| Sodium bisulfite | 10.0g |
| Isostearamidopropyl ethyl dimonium ethosulfate | 0.5g |
| 1-hydroxyethyl-5,5-dimethyl-2,4-imidazolidinedione | 0.3g |
| Acetate buffer* | 20.0g |
| Ascorbic acid | 0.5g |
| Deionized water | q.s. 100 g |

*Acetate buffer:

| | |
|---|---|
| Sodium acetate | 220g |
| Glacial acetic acid | 20ml |
| Deionized water | q.s. 1 liter |

Formulation is carried out by first dissolving the cationic surfactant in 90% of the deionized water with stirring. Next is added the acetate buffer and antioxidant with stirring followed by the sodium bisulfite. After all materials are dissolved, the pH is adjusted to 5.3 with 10% ammonium hydroxide after which water is added in a quantity sufficient (q.s.) to bring the composition to weight.

EXAMPLE II

Natural brown hair was given a permanent wave using the composition of Example I as follows: Hair was shampooed once with a mild acidic cleansing shampoo, rinsed thoroughly and toweled dry. While the hair may be slightly wet or dry, the moisture content should be even. Solution was applied directly to one section of the hair at a time with combing through for even distribution. Small, thoroughly saturated sections of hair were then wound on mandrels. After all sections had been wound on rods, each section was resaturated with solution. Air preheated to between 40° and 50°C was applied to the hair. Processing required between 15 and 30 minutes. After processing, the hair was rinsed with warm running water while still on the mandrels for at least 5 minutes. A towel was used to blot dry the hair and the hair allowed to rest several minutes before the rods were removed to give a natural looking soft wave style.

A curly hair style can be obtained by drying the hair with heat before removing the rods. Alternatively hair may be set on rollers in a conventional manner after rod removal.

EXAMPLE III

This example further illustrates the hair treating compositions of this invention.

TABLE II

| Ingredient | Amounts |
| --- | --- |
| Sodium bisulfite | 10.0g |
| Isostearamidopropyl ethyl dimonium ethosulfate | 0.5g |
| Dihydroxyethyl tallow glycinate | 0.5g |
| 1-hydroxyethyl-5,5-dimethyl-2,4-imidazolidinedione | 0.5g |
| Acetate buffer* | 10.0g |
| Erythorbic acid | 0.5g |
| Deionized water | q.s. 100 g |

*Acetate buffer:

| | |
| --- | --- |
| Sodium acetate | 220g |
| Glacial acetic acid | 20ml |
| Deionized water | q.s. 1 liter |

The ingredients in Table II are combined in the manner noted in Example I, ammonium hydroxide (10%) being used to adjust the pH to 5.3 as needed.

EXAMPLE IV

This example further illustrates a hair treating composition within the teachings of this invention.

TABLE III

| Ingredient | Amounts |
|---|---|
| Sodium bisulfite | 10.0g |
| Isostearamidopropyl ethyl dimonium ethosulfate | 0.5g |
| 1,3-dihydroxyethyl-5,5-dimethyl-2,4-imidazolidinedione | 0.5g |
| Ammonium acetate | 1.0g |
| Deionized water | q.s. 100g |

After formulating the components according to the method in Example I, 10% ammonium hydroxide is used to adjust the pH to 5.3 before bringing the solution to weight.

CLAIMS:

1. An aqueous hair treating composition which comprises:

a. a water-soluble bisulfite salt;

b. a water-soluble alkyl substituted betaine or quaternized amido amine derived cationic surfactant penetrating agent;

c. an imidazolidinedione having the formula:

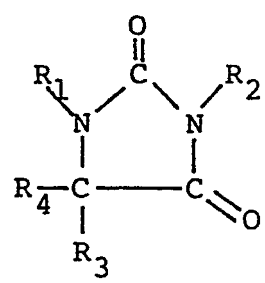

wherein $R_1$ and $R_2$ are the same or different and are hydrogen or hydroxyalkyl of 1 to 4 carbon atoms; $R_3$ and $R_4$ are the same or different and are hydrogen alkyl of 1 to 4 carbon atoms or phenyl; and the cosmetologically acceptable acid addition salts thereof;

d. a water-soluble organic acid salt; said composition being maintained at a pH between 5 and 6.5.

2. The composition according to claim 1, comprising:

a. said bisulfite salt in amount between 5.0 and 15.0 % (w/w);

b. said penetrating agent in an amount between 0.10 and 3.0 % (w/w);

c. said imidazolidinedione in an amount between 0.01 and 5 % (w/w);

d. said organic acid salt in an amount between 0.5 and 20.0 % (w/w); and

cosmetologically acceptable acid addition salt thereof in an amount of 0.1 to 3.0% (w/w);

d. said organic salt is sodium acetate in an amount of 1.0 to 10.0% (w/w); and

e. water is present in a quantity sufficient to make 100 percent.

5. The composition according to claim 3, wherein:

a. said bisulfite salt is sodium bisulfite in an amount of 10% (w/w);

b. said penetrating agent is bis-(2-hydroxyethyl) tallow ammonium ethanoate in an amount of 0.25 to 1.5% (w/w);

c. said imidazolidinedione is 1-hydroxymethyl-5,5-dimethyl-2,4-imidazolidinedione or a cosmetologically acceptable acid addition salt thereof in an amount of 0.1 to 3.0% (w/w);

d. said organic salt is sodium acetate in an amount of 1.0 to 10.0% (w/w); and

e. water is present in a quantity sufficient to make 100%.

6. The composition according to claim 2, wherein:

a. said bisulfite salt is sodium, potassium, ethanolamine or ammonium bisulfite;

b. said penetrating agent is a quaternary amido amine derived surfactant of the formula

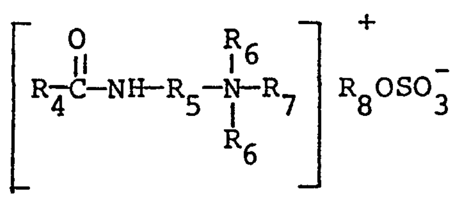

(B)

wherein $R_4$ is a saturated or unsaturated acid hydrocarbon radical of 12 to 20 carbons and $R_5$ through $R_8$ are alkyl radicals of 1-4 carbon atoms or hydrogen;

e. water in a quantity sufficient to make 100 percent.

3. The composition according to claim 2, wherein:

a. said bisulfite salt is sodium, potassium, ethanolamine or ammonium bisulfite;

b. said penetrating agent is an alkyl substituted betaine derived surfactant of the formula:

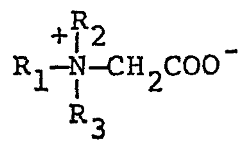

(A)

wherein $R_1$ is a saturated or unsaturated long chain fatty acid radical of 12 to 20 carbon atoms and $R_2$ and $R_3$ represent a short straight or branched chain alkyl or hydroxy alkyl radical of 1 to 4 carbon atoms;

c. said imidazolidinedione is a compound wherein $R_3$ and $R_4$ are alkyl of 1 to 4 carbon atoms and $R_1$ and $R_2$ are the same or different and are hydrogen or hydroxyalkyl of 1 to 4 carbon atoms or a cosmetologically acceptable acid addition salt thereof; and

d. said organic acid salt is then ammonium or alkaline metal acetate; said composition being maintained at a pH between 5.3 and 5.5.

4. The composition according to claim 3, wherein:

a. said bisulfite salt is sodium bisulfite in an amount of 10% (w/w);

b. said penetrating agent is bis-(2-hydroxyethyl) tallow ammonium ethanoate in an amount of 0.25 to 1.5% (w/w);

c. said imidazolidinedione is 1,3-dihydroxymethyl-5,5-dimethyl-2,4-imidazolidinedione or a

c. said imidazolidinedione is a compound wherein $R_3$ and $R_4$ are the same alkyl radical of 1-4 carbon atoms and $R_1$ and $R_2$ are the same or different and are hydrogen or hydroxyalkyl of 1-4 carbon atoms or a cosmetologically acceptable acid addition salt thereof; and

d. said organic acid salt is an ammonium or alkali metal acetate; said composition being maintained at a pH between 5.3 and 5.5.

7. The composition according to claim 6, wherein:

a. said bisulfite salt is sodium bisulfite in an amount of 10% (w/w);

b. said penetrating agent is N,N-dimethyl-n-ethyl-N-(3-isostearyl)- amidopropyl ammonium ethyl sulfate in an amount of 0.25 to 1.5% (w/w);

c. said imidazolidinedione is 1,3-dihydroxymethyl-5,5-dimethyl-2,4-imidazolidinedione or a cosmetologically acceptable acid addition salt thereof in an amount of 0.1 to 3.0% (w/w);

d. said organic acid salt is sodium acetate in an amount of 1.0-10.0% (w/w); and

e. water is present in quantity sufficient to make 100 percent.

8. The composition according to claim 6, wherein:

a. said bisulfite salt is sodium bisulfite in an amount of 10% (w/w);

b. said penetrating agent is N,N-dimethyl-N-ethyl-N-(3-isostearyl)-amido propyl ammonium ethyl sulfate in an amount of 0.25 to 1.5% (w/w);

c. said imidazolidinedione is 1-hydroxymethyl-5,5-dimethyl-2,4-imidazolidinedione or a cosmetologically acceptable acid addition salt thereof in an amount of 0.1 to 3.0% (w/w);

d. said organic acid salt is sodium acetate in an amount of 1.0-10.0% (w/w); and

e. water is present in a quantity sufficient to make 100 percent.

9. A process for treating hair which process comprises applying to the hair an aqueous composition according to any one of the preceding claims, shaping the hair, applying low or moderate heat for a time sufficient to effect hair treatment, and rinsing the hair with water.